(19) Europäisches Patentamt
European Patent Office
Office européen des brevets

(11) EP 0 523 168 B1

(12) EUROPEAN PATENT SPECIFICATION

(45) Date of publication and mention of the grant of the patent: **07.02.1996 Bulletin 1996/06**

(21) Application number: **91908040.8**

(22) Date of filing: **29.03.1991**

(51) Int. Cl.$^6$: **A61K 7/38**, C07C 31/34, A61K 7/32, A61K 7/48, C07D 493/04

(86) International application number: **PCT/US91/02062**

(87) International publication number: **WO 91/15191 (17.10.1991 Gazette 1991/24)**

# (54) GEL STICK ANTIPERSPIRANT COMPOSITIONS

SCHWEISSHEMMENDE GELSTIFTZUSAMMENSETZUNGEN

COMPOSITIONS POUR BATONS DE GEL ANTITRANSPIRATION

(84) Designated Contracting States: **DE FR GB IT**

(30) Priority: **06.04.1990 US 505807**

(43) Date of publication of application: **20.01.1993 Bulletin 1993/03**

(73) Proprietor: **THE PROCTER & GAMBLE COMPANY Cincinnati, Ohio 45202 (US)**

(72) Inventors:
- **OH, Young, Sik Fairfield, OH 45014 (US)**
- **JUNEJA, Prem, Sagar Cincinnati, OH 45242 (US)**
- **CONNOR, Daniel, Stedman Cincinnati, OH 45251 (US)**

(74) Representative: **Brooks, Maxim Courtney et al Newcastle-upon-Tyne NE12 9TS (GB)**

(56) References cited:

**EP-A- 0 051 681**
**EP-A- 0 319 917**
**US-A- 4 518 582**
**US-A- 4 664 909**
**US-A- 4 720 381**
**US-A- 4 722 835**
**US-A- 4 725 430**
**US-A- 4 743 444**
**US-A- 4 781 917**
**US-A- 4 996 334**

- **CHEMICAL ABSTRACTS, issued 21 August 1989, YUKIHIRO OHASHI et al., "Cosmetics Containing fluorinated dibenzylidene polyalcohol derivatives and nonaqueous gelation agents", see lines 6 to 14.**
- **CHEMICAL ABSTRACTS, issued 22 October 1990, "1,3:24-Di-O-benzylidene-D-sorbitol ethers as emulsion base for cosmetics, see entire article**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 523 168 B1

## Description

The present invention relates to antiperspirant compositions in the form of solid gel sticks. These compositions are stable while providing excellent antiperspirant performance to the user.

There are three main types of antiperspirant stick formulations: compressed powder sticks, gel sticks, and wax sticks. While each of these formulation types may have advantages in certain usage situations, each also has disadvantages. For example, compressed powder sticks are often brittle and hard, leaving a cosmetically-unacceptable powder on the skin upon application. Wax-based formulations can also yield cosmetically-unacceptable products due to such factors as hardness, greasiness, and stickiness. The opacity of such wax sticks and the residue created by their use may also be aesthetically undesirable.

Gel-based sticks have several advantages over both compressed powder and wax sticks. For example, the gel sticks tend to leave little or no residue or dust on the skin. Gel sticks also provide a vehicle which glides easily over the skin's surface resulting in very easy and comfortable application of the product.

Unfortunately, the formulation of antiperspirant compositions in the form of effective and stable gel sticks is difficult. The inclusion of antiperspirant actives in such compositions results in the final product having an acidic pH. Many of the gelling agents typically used in forming cosmetic gel sticks either do not form effective hard gels or tend to degrade under such acidic conditions, particularly in the presence of water. See for example US-A-4,720,381 The products sometimes degrade in periods as short as one month or less. For example, dibenzylidene sorbitol (DBS) is a well-known gelling agent. See, for example, US-A-4,154,816, Roehl et al., issued May 15, 1979; US-A-4,816,261, Luebbe et al., issued March 28, 1989; and US-A-4,743,444, McCall, issued May 10, 1988. Unfortunately, when DBS is used as a gelling agent in an acidic environment, such as is required in an antiperspirant product, it tends to degrade rapidly, resulting in a product which has a short shelf life. Since typical commercial distribution channels for antiperspirant products require storage for relatively long periods of time in factories, warehouses, and retail outlets, sometimes at elevated temperatures, degradation of the gelling agent and the resulting short product shelf life is a significant disadvantage. See, for example, US-A-4,518,582, Schamper et al., issued May 21, 1985. It, therefore, would be beneficial to identify a gelling agent which forms effective hard gels in an antiperspirant context, and is stable under the acidic conditions present in such products.

The effect that various substituents have upon the hydrolysis rates of some acetals and ketals is demonstrated in Kreevoy and Taft, J. Am. Chem. Soc., 77 5590-95 (1955).

Japanese Published Application 64-62377, Kao, published March 8, 1989, describes fluorinated dibenzylidene polyhydric alcohol derivatives which are effective gelling agents for cosmetic compositions containing a wide range of organic solvents. These compounds are not disclosed for use in antiperspirant compositions.

US-A-4,429,140, Murai et al., issued January 31, 1984, discloses a method for producing DBS and its derivatives. Disclosed DBS derivatives include those where the benzene ring is substituted with from 1 to 3 lower alkyl groups, lower alkoxy groups, halogen atoms or nitro groups. Para-chloro dibenzylidene xylitol and para-methoxy DBS are specifically disclosed; meta-substituted DBS derivatives are not taught. There is no suggestion to use these compounds in antiperspirant compositions.

US-A-4,371,645, Mahaffey, issued February 1, 1983, describes plastic compositions which include DBS derivatives for improved transparency. These DBS derivatives must include a chlorine or bromine substituent in the meta and/or para positions and may also include lower alkyl, hydroxy, methoxy, mono- or dialkyl amino, or fluorine substituents. Di(para-chloro) DBS, di(para-fluoro) DBS, and di(para-methoxy) DBS are all specifically disclosed. These compounds are not taught for use in antiperspirant compositions.

EP-A-0286522, Roquette Freres, published December 1, 1988, describes a process for making high purity alditol diacetals. Para-chloro DBS is disclosed. However, the use of such compounds in antiperspirant compositions is not suggested.

While the art teaches some DBS-type compounds derivatized with electron withdrawing groups, there is no teaching that such compounds must be derivatized at the meta position or of using such compounds in antiperspirant stick compositions. Further, there is no suggestion that such compounds, when used in the acidic environment of an antiperspirant stick formulation, would provide effective gels and long term stability.

The present invention provides very specific gelling agents which, when used in acidic antiperspirant stick formulations, provide good gel properties without the degradation problem which typically accompanies the use of DBS in antiperspirant sticks.

The present invention provides for solid antiperspirant compositions in gel stick form, having acidic pHs, comprising:

(a) from 0.5% to 35% by weight of an antiperspirant active;
(b) from 0.5% to 98% by weight of a solvent for said antiperspirant active; and
(c) from 0.5% to 10% by weight of a gelling agent selected from the group consisting of substituted dibenzylidene alditols (such as sorbitols, xylitols, and ribitols), and mixtures thereof, wherein at least one of the substituents on the benzene ring is selected from the group consisting of:

(1) $-CH_2F$, $-CH_2Cl$, -F, -Cl, -Br, -I, and $-CH=CHNO_2$, wherein at least one of these substituents is located at the meta position; and

(2) $-NO_2$, $-\overset{+}{N}H_3$, $-\overset{+}{N}R_3$, $-\overset{+}{P}R_3$, $-\overset{+}{P}H_3$, $-\overset{+}{S}R_2$, $-CF_3$, $-CCl_3$, $-CHF_2$, - $CHCl_2$, -CHClF, $-CCl_2F$, $-CF_2Cl$, $-SO_3H$, $-SO_3R$, $-CO_2H$, $-CO_2R$, $-CONH_2$, -CHO, -COR, and -C≡N, wherein R is $C_1$-$C_4$ alkyl and at least one of these substituents is located at the meta or para position.

Preferred gelling agents are substituted dibenzylidene sorbitols, particularly those which are substituted at the meta position with fluorine or chlorine.

The solid antiperspirant compositions encompassed by the present invention are in the form of gel sticks. These sticks have a suitable hardness such that they deposit an effective amount of antiperspirant material on the skin during normal use, while maintaining dimensional stability upon use and storage. Hardness of sticks can be determined by a variety of methods, including American Society for Testing and Materials (ASTM) Method D-5. This method involves the use of a needle or polished cone of particular weight and dimension, which is allowed to travel downward through the stick material for a predetermined period of time. The distance travelled by the needle or cone is a relative measure of stick hardness. Using Method D-5, with an ASTM-D1321 arrowhead-type penetration needle (Model 13-401-10, sold by Fischer Scientific Co.), weighing 50 grams, and a Model 13-399-10 Penetrometer (sold by Fischer Scientific Co.), the stick compositions of the present invention preferably have an average penetration value of from 60 to 200 millimeters, more preferably from 100 to 160 millimeters, over a period of 5 seconds. These values represent an average penetration for sticks within a given production batch, since such penetration values may vary from stick to stick within the batch.

The stick compositions of the present invention, by virtue of their incorporation of antiperspirant actives, are acidic in nature. Specifically, they have an apparent pH of from 1.5 to 4. The term "apparent pH" is used herein since the compositions are generally non-aqueous and, therefore, the pH of the composition is being measured in a non-aqueous system. Specifically, the pH is determined by melting the stick and measuring its pH at 25°C using a standard pH meter. If the stick is melted at a relatively high temperature (for example, about 120°C for about 1 hour), it will not resolidify upon cooling and the pH at 25°C can be easily measured. Under these conditions, the apparent pH of the compositions of the present invention should be from 1.5 to 4.

All parts, percentages and ratios specified herein are by weight, unless otherwise specified.

The required, as well as the optional, components of the present invention are described in detail below.

The compositions of the present invention include from 0.5% to 10%, preferably from 2% to 5%, most preferably from 2% to 3.5% by weight, of a specifically defined gelling agent component. This gelling agent component is a dibenzylidene alditol (for example, a sorbitol, xylitol or ribitol) substituted on the benzene ring at certain positions with one or more specific electron withdrawing groups. Dibenzylidene sorbitol (DBS) derivatives are preferred for use herein.

When the following substituents are utilized, at least one of the substituents must be located at the meta position of the benzene ring. Multiple substituents (including those not on the list) may be utilized as long as at least one from the list is located at the meta position. These substituents include: $-CH_2F$, $-CH_2Cl$, -F, -Cl, -Br, -I, and $-CH=CHNO_2$.

When the following substituents are utilized in the gelling agents, at least one of these substituents must be located at the meta or para position of the benzene ring. Once again, more than one of these substituents (or even substituents not on the list) may be included in a particular molecule, as long as at least one from the list is positioned at the meta or para position. These substituents include:

$-NO_2$, $-\overset{+}{N}H_3$, $-\overset{+}{N}R_3$, $-\overset{+}{P}R_3$, $-\overset{+}{P}H_3$, $-\overset{+}{S}R_2$, $-CF_3$, $-CCl_3$, $-CHF_2$, $-CHCl_2$, - CHClF, $-CCl_2F$, $-CF_2Cl$, $-SO_3H$, $-SO_3R$, $-CO_2H$, - $CO_2R$, $-CONH_2$, -CHO, -COR, and -C≡N, wherein R is $C_1$-$C_4$ alkyl.

Preferred gelling agents for use in the present invention include the following substituents at the meta position: - $CH_2F$, $-CH_2Cl$, -F, -Cl, -Br, -I, and $-CH=CHNO_2$; particularly preferred are the -F and -Cl substituents. It is preferred that this meta substitution be the only substitution on the benzene ring. The gelling agents have benzene rings at opposite ends of the molecule. The substituents described herein will generally be found on both benzene rings of the compound. Particularly preferred are di(meta-fluoro) dibenzylidene sorbitol and di(meta-chloro) dibenzylidene sorbitol.

To aid in understanding the present invention, the following are diagrams of dibenzylidene sorbitol, and dibenzylidene xylitol with the ortho, meta and para positions indicated.

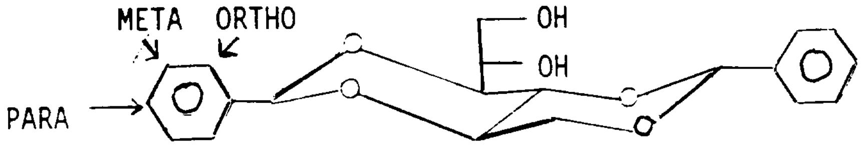


dibenzylidene sorbitol

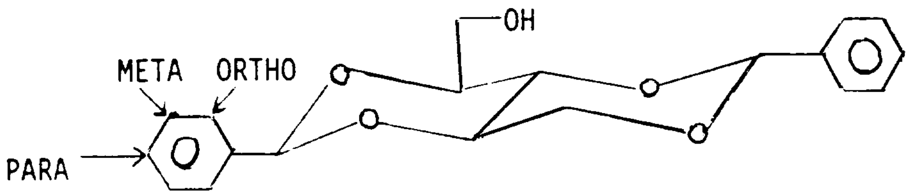


dibenzylidene xylitol

Dibenzylidene ribitol is structurally similar to dibenzylidene xylitol, except it is based on the sugar ribitol, rather than xylitol.

The structural formulas for di(meta-fluoro) DBS and di(meta-chloro) DBS are given below.

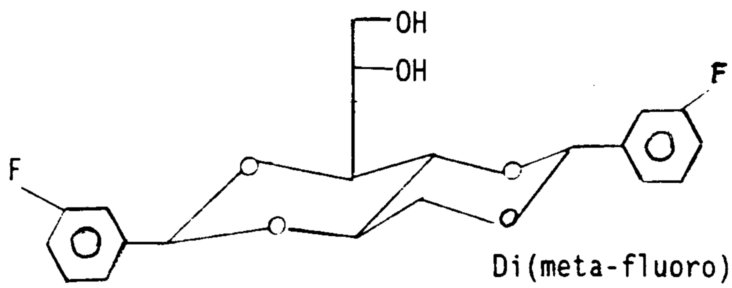

Di(meta-fluoro) DBS

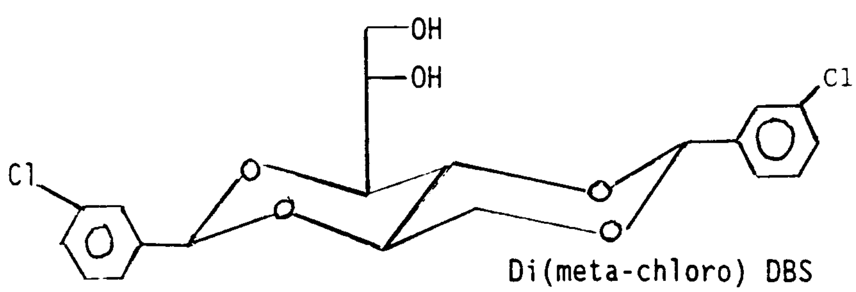

Di(meta-chloro) DBS

The substituents listed above provide gelling agents which exhibit good stability in the acid environment of an antiperspirant composition. A preferred sub-group of these substituents are those which have smaller molecular sizes since they tend to generally provide stronger gels. Thus, for example, the gel provided by a fluorine or chlorine substituted compound tends to be stronger than one provided by a trifluoromethane substituted compound. It is also preferred that the gelling agents utilized in the compositions of the present invention have a high purity. For example, they should be substantially free of para-toluene sulfonic acid or any other catalyst used in their synthesis as well as any salt forms (e.g., sodium) of these gelling agents. The presence of such impurities may tend to weaken the gel formed.

Mixtures of the gelling agents specified herein may be used in the compositions of the present invention.

In general, the gelling agents used in the present invention are formed by converting a meta-substituted benzaldehyde to the corresponding meta-substituted DBS using a reaction such as that taught in EP-A-0286522, Roquette Freres,

published December 1, 1988. As specific examples, the synthesis of meta-fluoro DBS and meta-chloro DBS is described below.

A solution of D-sorbitol (1006 g; 5.52 mol) in 3000 mL of distilled water, m-fluorobenzaldehyde (1240 g; 9.99 mol), and p-toluenesulfonic acid monohydrate (1310 g; 6.87 mol) is stirred at 30°C for 21 h. The resulting suspension is neutralized to a pH of 7.0-7.5 with an aqueous 10% NaOH solution, and the white precipitate is collected by filtration. The solid is then suspended and stirred, in succession, in reagent grade acetone (3 x 10.0 L), and hot (60°C) distilled water (3 x 10.0 L), collected, and dried *in vacuo* at 50°C to give 1113 g (47%) of purified di(meta-fluoro) DBS.

Di(meta-chloro) DBS is synthesized using a similar procedure, except that meta-chloro benzyaldehyde is used in place of meta-fluoro benzaldehyde.

Para-substituted compounds used in the present invention are synthesized using a similar procedure, except that para-substituted benzyaldehyde is utilized as the starting material. The general method for synthesizing substituted dibenzylidene xylitols and substituted dibenzylidene ribitols is taught in JP-A-64-62377, Kao, published March 8, 1989, and US-A 4,429,140, Murai et al., issued January 31, 1984, both.

The compositions of the present invention also contain from 0.5% to 35%, preferably from 5% to 25% by weight, of an antiperspirant active. This active may be incorporated either in solublized or particulate form. These weight percentages are calculated on an anhydrous metal salt basis (exclusive of glycine, the salts of glycine, or other complexing agents). If used in particulate form, the material preferably has a particle size of from 1 to 100 microns, preferably from 1 to 50 microns. They may be impalpable or microspherical in form and, preferably, have a high bulk density (e.g., greater than 0.7 g/cm$^3$). Such materials include, for example, many aluminum or zirconium astringent salts or complexes and are well known in the antiperspirant art.

Any aluminum astringent antiperspirant salt or aluminum and/or zirconium astringent complex can be employed herein. Salts useful as astringent antiperspirant salts or as components of astringent complexes include aluminum halides, aluminum hydroxyhalides, zirconyl oxyhalides, zirconyl hydroxyhalides, and mixtures of these materials.

Aluminum salts of this type include aluminum chloride and the aluminum hydroxyhalides having the general formula $Al_2(OH)_xQ_y.XH_2O$ where Q is chlorine, bromine or iodine; where x is from about 2 to about 5, and x+y = about 6 , and x and y do not need to be integers; and where X is from about 1 to about 6. Aluminum salts of this type can be prepared in the manner described more fully in US-A-3,887,692, Gilman, issued June 3, 1975, and US-A-3,904,741, Jones and Rubino, issued September 9, 1975.

The zirconium compounds which are useful in the present invention include both the zirconium oxy salts and zirconium hydroxy salts, also referred to as the zirconyl salts and zirconyl hydroxy salts. These compounds may be represented by the following general empirical formula:

$$ZrO(OH)_{2-nz}B_z$$

wherein z may vary from about 0.9 to about 2 and need not be an integer, n is the valence of B, 2-nz is greater than or equal to 0, and B may be selected from the group consisting of halides, nitrate, sulfamate, sulfate, and mixtures thereof. Although only zirconium compounds are exemplified in this specification, it will be understood that other Group IVB metal compounds, including hafnium, can be used in the present invention.

As with the basic aluminum compounds, it will be understood that the above formula is greatly simplified and is intended to represent and include compounds having coordinated and/or bound water in various quantities, as well as polymers, mixtures and complexes of the above. As will be seen from the above formula, the zirconium hydroxy salts actually represent a range of compounds having various amounts of the hydroxy group, varying from about 1.1 to only slightly greater than 0 groups per molecule.

Several types of antiperspirant complexes utilizing the above antiperspirant salts are known in the art. For example, US-A-3,792,068, Luedders et al., issued February 12, 1974, discloses complexes of aluminum, zirconium and amino acids, such as glycine. Complexes such as those disclosed in the Luedders et al. patent and other similar complexes are commonly known as ZAG. ZAG complexes are chemically analyzable for the presence of aluminum, zirconium and chlorine. ZAG complexes useful herein are identified by the specification of both the molar ratio of aluminum to zirconium (hereinafter "Al:Zr" ratio) and the molar ratio of total metal to chlorine (hereinafter "Metal:Cl" ratio). ZAG complexes useful herein have an Al:Zr ratio of from about 1.67 to about 12.5 and a Metal:Cl ratio of from about 0.73 to about 1.93.

Preferred ZAG complexes are formed by

(A) co-dissolving in water

(1) one part $Al_2(OH)_{6-m}Q_m$, wherein Q is an anion selected from the group consisting of chloride, bromide and iodide, and m is a number from about 0.8 to about 2.0;

(2) x parts $ZrO(OH)_{2-a}Q_a.nH_2O$, where Q is chloride, bromide or iodide; where a is from about 1 to about 2; where n is from about 1 to about 8; and where x has a value of from about 0.16 to about 1.2;

(3) p parts neutral amino acid selected from the group consisting of glycine, dl-tryptophane, dl-β-phenyl-alanine, dl-valine, dl-methionine and β-alanine, and where p has a value of from about 0.06 to about 0.53;

(B) co-drying the resultant mixture to a friable solid; and
(C) reducing the resultant dried inorganic-organic antiperspirant complex to particulate form.

A preferred aluminum compound for preparation of such ZAG type complexes is aluminum chlorhydroxide of the empirical formula $Al_2(OH)_5Cl.2H_2O$. Preferred zirconium compounds for preparation of such ZAG-type complexes are zirconyl hydroxychloride having the empirical formula $ZrO(OH)Cl.3H_2O$ and the zirconyl hydroxyhalides of the empirical formula $ZrO(OH)_{2-a}Cl_2.nH_2O$ wherein a is from about 1.5 to about 1.87, and n is from about 1 to about 7. The preferred amino acid for preparing such ZAG-type complexes is glycine of the formula $CH_2(NH_2)COOH$. Salts of such amino acids can also be employed in the antiperspirant complexes. See US-A-4,017,599, Rubino, issued April 12, 1977.

A wide variety of other types of antiperspirant complexes are also known in the art. For example, US-A-3,903,258, Siegal, issued September 2, 1975, discloses a zirconium aluminum complex prepared by reacting zirconyl chloride with aluminum hydroxide and aluminum chlorhydroxide. US-A-3,979,510, Rubino, issued September 7, 1976, discloses an antiperspirant complex formed from certain aluminum compounds, certain zirconium compounds, and certain complex aluminum buffers. US-A-3,981,896, issued September 21, 1976, discloses an antiperspirant complex prepared from an aluminum polyol compound, a zirconium compound and an organic buffer. US-A-3,970,748, Mecca, issued July 20, 1976, discloses an aluminum chlorhydroxy glycinate complex of the approximate general formula $[Al_2(OH)_4Cl][H_2CNH_2COOH]$.

Of all the above types of antiperspirant actives, preferred compounds include the 5/6 basic aluminum salts of the empirical formula $Al_2(OH)_5Cl.2H_2O$; mixtures of $AlCl_3.6H_2O$ and $Al_2(OH)_5Cl.\ 2H_2O$ with aluminum chloride to aluminum hydroxychloride weight ratios of up to about 0.5; ZAG type complexes wherein the zirconium salt is $ZrO(OH)Cl.3H_2O$, the aluminum salt is $Al_2(OH)_5Cl.\ 2H_2O$ or the aforementioned mixtures of $AlCl_3.6H_2O$ and $Al_2(OH)_5\ Cl.2H_2O$ wherein the total metal to chloride molar ratio in the complex is less than about 1.25 and the Al:Zr molar ratio is about 3.3, and the amino acid is glycine; and ZAG-type complexes wherein the zirconium salt is $ZrO(OH)_{2-a}Cl_a.nH_2O$ wherein a is from about 1.5 to about 1.87 and n is from about 1 to about 7, the aluminum salt is $Al_2(OH)_5Cl.2H_2O$, and the amino acid is glycine.

The aluminum chlorhydrate (ACH) actives are particularly preferred for use in the present invention since they tend to leave less residue than other actives when applied to skin.

Solubilized antiperspirant actives which may be utilized in the present invention are also well known in the art. These materials utilize monohydric or polyhydric alcohols or water to solubilize the antiperspirant active before it is incorporated into the product. Examples of such actives are taught, for example, in US-A-4,137,306, Rubino, issued January 30, 1979, filed June 23, 1989, and EP-A-0295070, published December 14, 1988. Examples of preferred solubilized actives include improved efficacy ACH (IACH) or aluminum sesquichlorhydrate solubilized in propylene glycol or propylene glycol/water mixtures.

The compositions of the present invention also include from 5% to 98%, preferably from 7% to 90%, most preferably from 60% to 85% by weight, of a solvent for the antiperspirant active component. The solvent not only carries the antiperspirant active, but also forms the base matrix of the solid stick when combined with the gelling agent. As will be appreciated by those skilled in the art, the selection of a particular solvent will depend upon the characteristics of the stick desired. For example, the solvent may be selected to provide such cosmetic benefits as emolliency when applied to the skin. Solvents useful herein include, for example, lower monohydric alcohols, polyhydric alcohols, and mixtures thereof. Water may also be used as a solvent, but is preferably present at levels of no greater than 5% of the final composition. Examples of solvents which may be utilized in the present invention include liquid polyethylene glycols (e.g., diethylene glycol), liquid polypropylene glycols (e.g., dipropylene glycol, tripropylene glycol), liquid polypropylene polyethylene glycol copolymers, water, ethanol, n-propanol, n-butanol, t-butanol, 2-methoxyethanol, 2-ethoxyethanol, ethylene glycol, 1,2-propylene glycol, 1,3-propylene glycol, 1,4-butylene glycol, 1,2-butylene glycol, isopropanol, isobutanol, diethylene glycol monomethylether, diethylene glycol monoethylether, 1,3-butylene glycol, 2,3-butylene glycol, 2,4-dihydroxy-2-methylpentane, trimethylene glycol, glycerine, 1,3-butane diol, 1,4-butane diol, hexylene glycol, and the like, and mixtures thereof. As used herein, polyethylene glycols, polypropylene glycols, and polypropylene polyethylene glycol copolymers include the alkoxylated (e.g., ethoxylated, propoxylated, butoxylated) versions of these compounds. Examples of such compounds are butoxylated polypropylene polyethylene glycol copolymers, such as PPG-5-buteth-7.

These solvents are fully described, for example, in US-A-4,518,582, Schamper et al., issued May 21, 1985, and EP-A-107,330, Luebbe et al., published May 2, 1984. The preferred solvents for use herein include liquid polyethylene glycols, liquid polypropylene glycols, liquid polypropylene polyethylene glycol copolymers, propylene glycol, 1,3-butylene glycol, and 2,4-dihydroxy-2-methylpentane (sometimes referred to as hexylene glycol), and mixtures thereof. Particularly preferred solvents include propylene glycol, dipropylene glycol, tripropylene glycol, hexylene glycol, and mixtures thereof.

The compositions of the present invention may also contain optional components which modify the physical characteristics of the compositions or serve as "active" components when deposited on the skin in addition to the antiperspirant material. Optional components useful herein are described in the following documents: US-A-4,049,792, Elsnau, issued September 20, 1977; CN-A-1,164,347, Beckmeyer et al., issued March 27, 1984; EP-A-0,117,070, May, published August 29, 1984; and Geria, "Formulation of Stick Antiperspirants and Deodorants", *Cosmetics & Toiletries, 99*:55-60 (1984).

The specific non-active components that may be useful will depend upon the characteristics desired for the particular stick composition. Such components include, for example, emollients, colorants, perfumes, and emulsifiers. Although the present compositions may contain fillers and particulate materials (such as talc, hydrophobic (quaternized) clay and silica (fumed and non-fumed)) other than the antiperspirant active described above, such particulates should be used sparingly inasmuch as they could leave a residue on the skin if used at too high a level. As used herein, "particulate materials" are those materials that neither dissolve in the composition components nor melt during the manufacture of the stick.

The compositions of the present invention may contain from 1% to 40% by weight of one or more emollients. These emollients may have an intermediate polarity, such as the ethyl, isopropyl and n-butyl diesters of adipic, phthalic and sebacic acids. Preferred examples of such emollients include di-n-butyl phthalate, diethyl sebacate, diisopropyl adipate and ethyl carbomethyl phthalate, all of which are disclosed in US-A-4,045,548, Luedders et al., issued August 30, 1977. Other useful emollients include $C_{12}$-$C_{15}$ alcohol benzoates (commercially available as Finsolv from Finetex, Inc.). The compositions of the present invention may also include non-polar emollients, such as volatile silicone oils, non-polar non-volatile emollients, and mixtures thereof. The term "volatile", as used herein, refers to those materials which have a measurable vapor pressure at ambient temperature.

Volatile silicone oils useful in the cosmetic stick compositions of the present invention are preferably cyclic or linear polydimethylsiloxanes containing from about 3 to about 9, preferably from about 4 to about 5, silicon atoms. The following formula illustrates cyclic volatile polydimethylsiloxanes useful in the antiperspirant stick compositions disclosed herein:

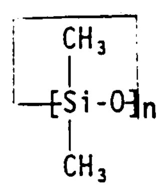

wherein n equals about 3 to about 7. Linear polydimethylsiloxanes contain from about 3 to about 9 silicon atoms per molecule and have the following general formula:

$$(CH_3)_3Si\text{-}O\text{-}[Si(CH_3)_2\text{-}O]_n\text{-}Si(CH_3)_3$$

wherein n equals about 1 to about 7. Linear volatile silicone materials generally have viscosities of less than about 0.05 $cm^2/s$ (5 centistokes) at 25°C, while cyclic materials typically have viscosities of less than about 0.1 $cm^2/s$ (10 centistokes). A description of various volatile silicone oils is found in Todd et al., "Volatile Silicone Fluids for Cosmetics", *Cosmetic & Toiletries, 91*, pages 27-32 (1976).

Examples of preferred volatile silicone oils useful herein include: Dow Corning 344, Dow Corning 345, and Dow Corning 200 (manufactured by Dow Corning Corp.); Silicone 7207 and Silicone 7158 (manufactured by Union Carbide Corp.); SF 1202 (manufactured by General Electric); and SWS-03314 (manufactured by SWS Silicones, Inc.).

Non-volatile silicone oils useful as emollient materials include polyalkylsiloxanes, polyarylsiloxanes and polyethersiloxane copolymers. The essentially non-volatile polyalkyl siloxanes useful herein include, for example, polydimethyl siloxanes with viscosities of from about 0.05 to about 1,000 $cm^2/s$ (about 5 to about 100,000 centistokes) at 25°C. Among the preferred non-volatile emollients useful in the present compositions are the polydimethyl siloxanes having viscosities from about 0.02 to about 4 $cm^2/s$ (about 2 to about 400 centistokes) at 25°C. Such polyalkyl siloxanes include the Viscasil series (sold by General Electric Company) and the Dow Corning 200 series (sold by Dow Corning Corp.). Polyalkylaryl siloxanes include polymethylphenyl siloxanes having viscosities of from 0.15 to 0.65 $cm^2/s$ (15 to 65 centistokes) at 25°C. These are available, for example, as SF 1075 methylphenyl fluid (sold by General Electric Company) and 556 Cosmetic Grade Fluid (sold by Dow Corning Corp.). Useful polyether siloxane copolymers include, for example, a polyoxyalkylene ether copolymer having a viscosity of 12 to 15 $cm^2/s$ (1200 to 1500 centistokes) at 25°C. Such a fluid is available as SF1066 organosilicone surfactant (sold by General Electric Company). Polysiloxane ethylene glycol ether copolymers are preferred copolymers for use in the present compositions.

The compositions of the present invention may contain from 1% to 15% by weight of a processing aid. These components act to reduce the gel formation temperature of the composition or reduce the melting temperature of the gellant. Examples of such materials are taught in US-A-4,719,102, Randhawa et al., issued January 12, 1988 and include,

among others, propylene carbonate, butyrolactone, caprolactone, and mixtures thereof. The compositions may also include from 0.1% to 1% by weight of a stabilizing agent which acts to stabilize the composition (especially the gelling agent) during the high temperature steps of the manufacturing process. Examples of these material include zinc acetate, methenamine, magnesium acetate, calcium acetate, triethanolamine, diethanolamine, and mixtures thereof.

The compositions of the present invention may also contain from about 0.5% to about 10% of an inert filler material. Suitable filler materials include talc, colloidal silica (such as Cab-O-Sil, sold by Cabot Corp.), clays (such as bentonite), hydrophobic (quaternized) clays, and mixtures thereof. The use of such fillers as stabilizing agents in cosmetic sticks is disclosed in US-A-4,126,679, Davy et al., issued November 21, 1987. The compositions of the present invention may also include perfumes, emulsifiers and coloring agents well known in the art, at levels of from 0.1% to 5% by weight.

In addition to the antiperspirant actives, discussed above, the antiperspirant sticks of the present invention may also contain a safe and effective amount of one or more additional components which are meant to be deposited upon human tissue. Such active components include astringents, bacteriostats, fungistats, pigments, dyes, colorants, perfumes, emollients, ultraviolet absorbers, and mixtures thereof. These components must be stable in the formulation of the instant invention. A "safe and effective" amount of such active components is that amount which yields the desired benefit at a reasonable benefit/risk ratio for human usage. Various active components among those useful in the present invention are described in US-A-4,226,889, Yuhas, issued October 7, 1980.

The compositions of this invention may be made by methods known to those skilled in the art. Such methods are described in "Gels and Sticks Formulary", *Cosmetics & Toiletries, 99*, 77-84 (1984). Such methods generally involve admixture of the DBS-derivative gelling agent to a solvent upon heating to a temperature sufficient to dissolve the gelling agent. Such temperature is generally from 110°C to 135°C. The antiperspirant active and optional components are then added and the solution is poured into stick molds. A solid gel forms upon cooling. As the stick composition may solidify rapidly upon cooling, care should be taken so as to maintain an elevated temperature while mixing and processing the composition.

The gel form antiperspirant stick compositions of the present invention are used in a conventional manner. Specifically, the compositions may be used to prevent and/or control perspiration wetness by topically applying, one or more times a day, an effective amount of the composition to areas of the body particularly prone to perspiration (e.g., the underarm or axillary area).

The following non-limiting examples illustrate the compositions, methods of making, and methods of use described in the present application.

EXAMPLES

Opaque antiperspirant gel stick compositions of the present invention, having the formulas given below, are made by the following procedure.

Phase A - Weigh the required amount of each solvent into a 3-neck round bottom flask containing a stir bar. Place the flask in a heating mantle connected to a rheostat.

Weigh the required amount of gellant and add it to the solvents in the flask. Connect a water-cooled condenser to one neck of the flask, place a thermometer in another neck and cover the 3rd neck with a stopper.

Heat the flask while stirring the contents until the gellant is completely solubilized (usually 110°C-132°C).

Add the clay and silica to the gellant solution. Remove the flask from the heating mantle but continue stirring the mixture. When the temperature of the mixture drops down to 102°C ± 3°C, add phase B to this mixture. Stir for no longer than 3 minutes then pour into canisters (cover each canister loosely). Allow to cool to room temperature.

Phase B - Weigh the glycol solvent into a beaker containing a mechanical stirrer. Add an equal amount of powdered antiperspirant active and let stir for at least 2 hours at room temperature before adding to phase A.

These antiperspirant stick compositions have an apparent pH of about 2.5.

| Ingredients | Examples | | |
|---|---|---|---|
| | I | II | III |
| Phase A | | | |
| Dipropylene glycol | 31.90 | 36.72 | 36.61 |
| Hexylene glycol | 20.03 | - | - |
| PPG-5-Buteth-7 | - | 19.91 | - |
| Tripropylene glycol | - | - | 19.81 |
| Propylene carbonate | 7.98 | 8.13 | 7.97 |
| Finsolv TN | 4.99 | - | - |
| Di(meta-fluoro) DBS | 2.50 | 2.50 | 2.48 |
| Bentone 38 [1] | 1.50 | 1.49 | 1.49 |
| Cabosil [3] | 1.00 | 1.00 | 1.00 |
| Phase B | | | |
| I-ACH [2]/Propylene glycol | 30.10 | 30.25 | - |
| (50/50) | - | - | |
| I-ACH/PEG-8 (50/50) | | | 30.64 |

[1] Commercially available from N L Industries
[2] Commercially available from Westwood
[3] Commercially available from Cabot Corp.

Substantially similar results are obtained when the di(meta-fluoro) DBS is replaced, in whole or in part, by an equivalent amount of di(meta-chloro) DBS.

The opaque sticks produced are storage stable and provide excellent antiperspirant efficacy when applied to the axillary area.

Clear antiperspirant gel stick compositions of the present invention, having the formulas given below, are made by the following procedure.

Phase A - Weigh the required amount of each solvent into a 3-neck round bottom flask containing a stir bar. Place the flask in a heating mantle connected to a rheostat.

Weigh the required amount of gellant and add it to the solvents in the flask. Connect a water-cooled condenser to one neck of the flask, place a thermometer in another neck and cover the 3rd neck with a stopper.

Heat the flask while stirring the contents until all the gellant dissolves (usually 110°C-132°C). After the gellant is completely solubilized, take the flask out of the heating mantle but continue stirring the solution. When the solution temperature drops down to 94°C ± 3°C, add phase B to it, stir for 2-3 min., but not more than about 5 min. Pour the mixture into canisters and cover loosely. Allow to cool to room temperature. Phase B - Dissolve the antiperspirant active in propylene glycol (PG) and water ($H_2O$) such that the resulting mixture is 50/46/4 I-ACH/PG/$H_2O$. Heat this mixture in a 3-neck round bottom flask using the same setup as phase A. Maintain the temperature at 55-60°C.

These antiperspirant stick compositions have an apparent pH of between about 1.5 and about 4.

| Ingredients | Examples | | | |
|---|---|---|---|---|
| | IV | V | VI | VII |
| Phase A | | | | |
| Tripropylene glycol | - | - | 6.50 | - |
| Dipropylene glycol | 34.36 | 33.97 | 29.95 | 29.40 |
| Hexylene glycol | - | 20.00 | | |
| PPG-5-buteth-7 [1] | 24.85 | - | 20.00 | 19.90 |
| Propylene carbonate | 8.02 | 8.01 | 8.10 | 7.97 |
| Finsolv TN (RTM) | - | 5.00 | - | - |
| PEG 400 | - | - | 3.12 | 10.03 |
| Di(meta-fluoro) DBS | 2.51 | 3.02 | 2.51 | 2.52 |
| Phase B | | | | |
| I-ACH [2] | 15.13 | 15.00 | 14.91 | 15.09 |
| Propylene glycol | 13.93 | 13.80 | 13.71 | 13.89 |
| Water | 1.20 | 1.20 | 1.20 | 1.20 |

[1] Commercially available from Union Carbide
[2] Commercially available from Westwood

Substantially similar results are obtained when the di(meta-fluoro) DBS is replaced, in whole or in part, by an equivalent amount of di(meta-chloro) DBS.

The clear sticks produced are storage stable and provide excellent antiperspirant efficacy when applied to the axillary area.

## Claims

1. A solid antiperspirant composition in gel stick form, having an acidic pH, characterized in that it comprises:

   (a) from 0.5% to 35% by weight of an antiperspirant active;
   (b) from 0.5% to 98% by weight, preferably from 7% to 90%, of a solvent for said antiperspirant active; and
   (c) from 0.5% to 10%, preferably from 2% to 5%, most preferably from 2% to 3.5% by weight, of a gelling agent selected from substituted dibenzylidene alditols and mixtures thereof, wherein at least one of the substituents on the benzene ring is selected from:

   (1) $-CH_2F$, $-CH_2Cl$, -F, -Cl, -Br, -I, and $-CH=CHNO_2$, wherein at least one of these substituents is located at the meta position; and
   (2) $-NO_2$, $-\overset{+}{N}H_3$, $-\overset{+}{N}R_3$, $-\overset{+}{P}R_3$, $-\overset{+}{P}H_3$, $-\overset{+}{S}R_2$, $-CF_3$, $-CCl_3$, $-CHF_2$, $-CHCl_2$, $-CHClF$, $-CCl_2F$, $-CF_2Cl$, $-SO_3H$, $-SO_3R$, $-CO_2H$, $-CO_2R$, $-CONH_2$, -CHO, -COR, and $-C{\equiv}N$, wherein R is $C_1$-$C_4$ alkyl and at least one of these substituents is located at the meta or para position.

2. A solid antiperspirant composition according to Claim 1 characterized in that the substituted dibenzylidene alditol is selected from substituted dibenzylidene sorbitols, substituted dibenzylidene xylitols, substituted dibenzylidene ribitols, and mixtures thereof, and is preferably a substituted dibenzylidene sorbitol.

3. A solid antiperspirant composition according to Claim 1 or 2 characterized in that, in the gelling agent, at least one of the substituents on the benzene ring is selected from $-CH_2F$, $-CH_2Cl$, -F, -Cl, -Br, -I, and $-CH=CHNO_2$, and at least one of these substituents is located at the meta position.

4. A solid antiperspirant composition according to any of Claims 1-3 characterized in that, in the gelling agent, the sole substituent on the benzene ring is selected from -F and -Cl.

5. A solid antiperspirant composition according to any of Claims 1-4 characterized in that the gelling agent is di(meta-fluoro) dibenzylidene sorbitol.

6. A solid antiperspirant composition according to any of Claims 1-5 characterized in that it has an apparent pH of from 1.5 to 4.

7. A solid antiperspirant composition according to any of Claims 1-6 characterized in that the solvent is selected from liquid polyethylene glycols, liquid polypropylene glycols, liquid polyethylene polypropylene glycol copolymers, water, ethanol, n-propanol, n-butanol, t-butanol, 2-methoxyethanol, 2-ethoxyethanol, ethylene glycol, 1,2-propylene glycol, 1,3-propylene glycol, 1,4-butylene glycol, 1,2-butylene glycol, isopropanol, isobutanol, diethylene glycol monomethylether, diethylene glycol monoethylether, 1,3-butylene glycol, 2,3-butylene glycol, 2,4-dihydroxy-2-methylpentane, trimethylene glycol, glycerine, 1,3-butane diol, 1,4-butane diol, hexylene glycol, and mixtures thereof, and is preferably selected from propylene glycol, dipropylene glycol, tripropylene glycol, hexylene glycol, and mixtures thereof.

8. A cosmetic method for preventing and controlling perspiration wetness in humans characterized in that it comprises the application to the underarm area of an effective amount of the solid antiperspirant composition according to any of Claims 1-7.

**Patentansprüche**

1. Feste schweißhemmende Zusammensetzung in Gelstiftform mit einem sauren pH, dadurch gekennzeichnet, daß sie

(a) 0,5 bis 35 Gew.-% eines schweißhemmenden Wirkstoffs;
(b) 0,5 bis 98 Gew.-%, vorzugsweise 7 bis 90 Gew.-%, eines Lösungsmittels für den schweißhemmenden Wirkstoff; und
(c) 0,5 bis 10, vorzugsweise 2 bis 5, weiter vorzugsweise 2 bis 3,5 Gew.-% eines Geliermittels, gewählt aus substituierten Dibenzylidenalditolen und Mischungen hiervon umfaßt, wobei mindestens einer der Substituenten an dem Benzolring aus

(1) $-CH_2F$, $-CH_2Cl$, -F, -Cl, -Br, -I, und $-CH=CHNO_2$, worin mindestens einer dieser Substituenten an der meta-Position angeordnet ist; und
(2) $-NO_2$, $-\overset{+}{N}H_3$, $-\overset{+}{N}R_3$, $-\overset{+}{P}R_3$, $-\overset{+}{P}H_3$, $-\overset{+}{S}R_2$, $-CF_3$, $-CCl_3$, $-CHF_2$, $-CHCl_2$, -CHClF, $-CCl_2F$, $-CF_2Cl$, $-SO_3H$, $-SO_3R$, $-CO_2H$, $-CO_2R$, $-CONH_2$, -CHO, -COR, und $-C{\equiv}N$, worin R $C_1$-$C_4$-Alkyl ist und mindestens einer dieser Substituenten an der meta- oder para-Position angeordnet ist, gewählt ist.

2. Feste schweißhemmende Zusammensetzung nach Anspruch 1, dadurch gekennzeichnet, daß das substituierte Dibenzylidenalditol aus substituierten Dibenzylidensorbitolen, substituierten Dibenzylidenxylitolen, substituierten Dibenzylidenribitolen und Mischungen hiervon gewählt ist und vorzugsweise ein substituiertes Dibenzylidensorbitol ist.

3. Feste schweißhemmende Zusammensetzung nach Anspruch 1 oder 2, dadurch gekennzeichnet, daß bei dem Geliermittel mindestens einer der Substituenten an dem Benzolring aus $-CH_2F$, $-CH_2Cl$, -F, -Cl, -Br, -I, und $-CH=CHNO_2$ gewählt ist und mindestens einer dieser Substituenten an der meta-Position angeordnet ist.

4. Feste schweißhemmende Zusammensetzung nach mindestens einem der Ansprüche 1 bis 3, dadurch gekennzeichnet, daß bei dem Geliermittel der einzige Substituent an dem Benzolring aus -F und -Cl gewählt ist.

5. Feste schweißhemmende Zusammensetzung nach mindestens einem der Ansprüche 1 bis 4, dadurch gekennzeichnet, daß das Geliermittel Di(meta-fluor)dibenzylidensorbitol ist.

6. Feste schweißhemmende Zusammensetzung nach mindestens einem der Ansprüche 1 bis 5, dadurch gekennzeichnet, daß sie einen scheinbaren pH von 1,5 bis 4 aufweist.

7. Feste schweißhemmende Zusammensetzung nach mindestens einem der Ansprüche 1 bis 6, dadurch gekennzeichnet, daß das Lösungsmittel aus flüssigen Polyethylenglykolen, flüssigen Polypropylenglykolen, flüssigen Polyethylenpolypropylenglykol-Copolymeren, Wasser, Ethanol, n-Propanol, n-Butanol, t-Butanol, 2-Methoxyethanol, 2-Ethoxyethanol, Ethylenglykol, 1,2-Propylenglykol, 1,3-Propylenglykol, 1,4-Butylenglykol, 1,2- Butylenglykol, Isopropanol, Isobutanol, Diethylenglykolmonomethylether, Diethylenglykolmonoethylether, 1,3-Butylenglykol, 2,3-Butylenglykol, 2,4-Dihydroxy-2-methylpentan, Trimethylenglykol, Glycerin, 1,3-Butandiol, 1,4-Butandiol, Hexylenglykol und

Mischungen hiervon gewählt ist und vorzugsweise aus Propylenglykol, Dipropylenglykol, Tripopylenglykol, Hexylenglykol und Mischungen hiervon gewählt ist.

8. Kosmetisches Verfahren zur Verhinderung und Regulierung der Schweißfeuchtigkeit bei Menschen, dadurch gekennzeichnet, daß es die Aufbringung einer wirksamen Menge der festen schweißhemmenden Zusammensetzung nach mindestens einem der Ansprüche 1 bis 7 auf den Unterarmbereich umfaßt.

**Revendications**

1. Composition solide antitranspiration sous forme de gel en stick, ayant un pH acide, caractérisée en ce qu'elle comprend:

   (a) de 0,5% à 35% en poids d'une substance active antitranspiration;
   (b) de 0,5% à 98% en poids, de préférence de 7% à 90%, d'un solvant pour ladite substance active antitranspiration; et
   (c) de 0,5% à 10%, de préférence de 2% à 5%, mieux encore de 2% à 3,5% en poids, d'un agent gélifiant choisi parmi les dibenzylidène alditols substitués et leurs mélanges, dans lesquels au moins un des substituants du cycle benzénique est choisi parmi:

      (1) $-CH_2F$, $-CH_2Cl$, -F, -Cl, -Br, -I, et $-CH{=}CHNO_2$, dans lesquels au moins un de ces substituants se trouve en position méta; et
      (2) $-NO_2$, $-\overset{+}{N}H_3$, $-\overset{+}{N}$, $-\overset{+}{P}R_3$, $-\overset{+}{P}H_3$, $-\overset{+}{S}R_2$, $-CF_3$, $-CCl_3$, $-CHF_2$, $-CHCl_2$, -CHClF, $-CCl_2F$, $-CF_2Cl$, $-SO_3H$, $-SO_3R$, $-CO_2H$, $-CO_2R$, $-CONH_2$, -CHO, -COR, et $-C{\equiv}N$, où R est un groupe alkyle en $C_1$-$C_4$ et au moins un de ces substituants se trouve en position méta ou para.

2. Composition solide antitranspiration selon la revendication 1, caractérisée en ce que le dibenzylidène alditol substitué est choisi parmi les dibenzylidène sorbitols substitués, les dibenzylidène xylitols substitués, les dibenzylidène ribitols substitués, et leurs mélanges, et est de préférence un dibenzylidène sorbitol substitué.

3. Composition solide antitranspiration selon la revendication 1 ou 2, caractérisée en ce que dans l'agent gélifiant, au moins un des substituants du cycle benzénique est choisi parmi $-CH_2F$, $-CH_2Cl$, -F, -Cl, -Br, -I, et $-CH{=}CHNO_2$, et au moins un de ces substituants se trouve en position méta.

4. Composition solide antitranspiration selon l'une quelconque des revendications 1-3, caractérisée en ce que dans l'agent gélifiant, le seul substituant sur le cycle benzénique est choisi parmi -F et -Cl.

5. Composition solide antitranspiration selon l'une quelconque des revendications 1-4, caractérisée en ce que l'agent gélifiant est le di(métafluoro)dibenzylidène sorbitol.

6. Composition solide antitranspiration selon l'une quelconque des revendications 1-5, caractérisée en ce qu'elle possède un pH apparent de 1,5 à 4.

7. Composition solide antitranspiration selon l'une quelconque des revendications 1-6, caractérisée en ce que le solvant est choisi parmi les polyéthylèneglycols liquides, les polypropylèneglycols liquides, les copolymères polyéthylène polypropylèneglycol liquides, l'eau, l'éthanol, le n-propanol, le n-butanol, le t-butanol, le 2-méthoxyéthanol, le 2-éthoxyéthanol, l'éthylèneglycol, le 1,2-propylèneglycol, le 1,3-propylèneglycol, le 1,4-butylèneglycol, le 1,2-butylèneglycol, l'isopropanol, l'isobutanol, l'éther monométhylique de diéthylèneglycol, l'éther monoéthylique de diéthylèneglycol, le 1,3-butylèneglycol, le 2,3-butylèneglycol, le 2,4-dihydroxy-2-méthylpentane, le triméthylèneglycol, la glycérine, le 1,3-butanediol, le 1,4-butanediol, l'hexylèneglycol, et leurs mélanges, et est de préférence choisi parmi le propylèneglycol, le dipropylèneglycol, le tripropylèneglycol, l'hexylèneglycol, et leurs mélanges.

8. Procédé cosmétique pour prévenir et lutter contre l'humidité de la transpiration chez l'homme, caractérisé en ce qu'il comprend l'application sur la surface des aisselles d'une quantité efficace de la composition solide antitranspiration selon l'une quelconque des revendications 1-7.